(19) Europäisches Patentamt
European Patent Office
Office européen des brevets

(11) Veröffentlichungsnummer: **0 161 442**
**A2**

# (12) EUROPÄISCHE PATENTANMELDUNG

(21) Anmeldenummer: **85103772.1**

(22) Anmeldetag: **28.03.85**

(51) Int. Cl.⁴: **C 07 D 277/48**, A 01 N 47/28

(30) Priorität: **12.04.84 DE 3413755**

(43) Veröffentlichungstag der Anmeldung: **21.11.85**
**Patentblatt 85/47**

(84) Benannte Vertragsstaaten: **BE CH DE FR GB IT LI NL**

(71) Anmelder: **BASF Aktiengesellschaft,**
**Carl-Bosch-Strasse 38, D-6700 Ludwigshafen (DE)**

(72) Erfinder: **Lange, Arno, Dr., Oberes Geistal 3b,**
**D-6702 Bad Duerkheim (DE)**
Erfinder: **Parg, Adolf, Dr., Paray-le-Monial-Strasse 8,**
**D-6702 Bad Duerkheim (DE)**
Erfinder: **Wuerzer, Bruno, Dr. Dipl.-Landwirt,**
**Ruedigerstrasse 13, D-6701 Otterstadt (DE)**

(54) **Thiazolylharnstoffe, Verfahren zu ihrer Herstellung und ihre Verwendung zur Bekämpfung unerwünschten Pflanzenwuchses.**

(57) Die Erfindung betrifft Thiazolylharnstoffe der Formel

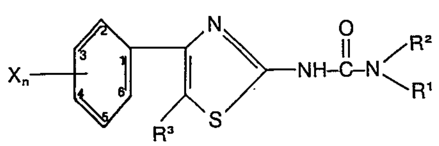

in der
$R^1$ Wasserstoff oder Alkyl,
$R^2$ Alkyl, Alkoxy, Alkenyl, Alkinyl oder Cycloalkyl,
$R^3$ Wasserstoff oder Alkyl,
X Wasserstoff, Halogen, Alkyl, Alkoxy, Halogenalkyl, Halogenalkoxy, Cycloalkyl, Alkylthio, Nitro, Cyano, Benzyloxy, gegebenenfalls substituiertes Phenyl oder Phenoxy und
n 1, 2 oder 3 bedeuten
Verfahren zu ihrer Herstellung und ihre Verwendung zur Bekämpfung unerwünschten Pflanzenwuchses.

EP 0 161 442 A2

Thiazolylharnstoffe, Verfahren zu ihrer Herstellung und ihre Verwendung zur Bekämpfung unerwünschten Pflanzenwuchses

Die vorliegende Erfindung betrifft Thiazolylharnstoffe, Verfahren zu ihrer Herstellung, Herbizide, welche diese Verbindungen enthalten, sowie Verfahren zur Bekämpfung unerwünschten Pflanzenwuchses mit diesen Verbindungen.

Es ist bekannt, daß Thiazolylharnstoffe, die in 4-Stellung am Heterocyclus gegebenenfalls durch Halogen, Alkyl, Alkoxy, Halogenalkyl oder Halogenalkoxy substituierte Arylreste tragen, herbizid wirksam sind. Als einzige Vertreter dieser Klasse sind jedoch nur Harnstoffe mit 4-(4-Chlorphenyl)- bzw. 4-(4-Methoxyphenyl)-thiazol-2-ylresten genannt. Diese Verbindungen sind nützlich zur Inhibierung des Wachstums oder zur Zerstörung verschiedener Fremdpflanzen, wie beispielsweise Gräsern, Buschwerk und unerwünschtem Gestrüpp (GB-A-1 131 207).

Es wurde gefunden, daß Thiazolylharnstoffe der Formel I

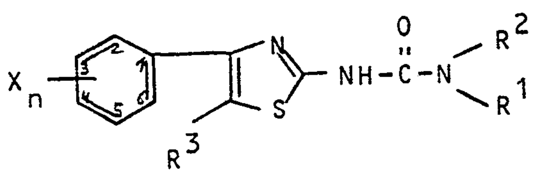 (I),

in der

$R^1$ Wasserstoff oder $C_1$-$C_4$-Alkyl,

$R^2$ $C_1$-$C_4$-Alkyl, $C_1$-$C_4$-Alkoxy, $C_2$-$C_4$-Alkenyl, $C_2$-$C_4$-Alkinyl oder $C_3$-$C_7$-Cycloalkyl,

$R^3$ Wasserstoff oder $C_1$-$C_4$-Alkyl,

X Wasserstoff, Halogen, $C_1$-$C_6$-Alkoxy, $C_1$-$C_6$-Halogenalkoxy, $C_1$-$C_6$-Alkyl, $C_1$-$C_6$-Halogenalkyl, $C_3$-$C_6$-Cycloalkyl, $C_1$-$C_6$-Alkylthio, Nitro, Cyano, Benzyloxy, gegebenenfalls durch Halogen substituiertes Phenyl oder gegebenenfalls durch Halogen und/oder Halogenmethyl substituiertes Phenoxy und

n 1, 2 oder 3 bedeuten,

mit der Maßgabe, daß X nicht Wasserstoff, Chlor oder Methoxy in 4-Position oder Chlor in 3- und 4-Position bedeutet, wenn $R^1$ Methyl, $R^2$ und $R^3$ Wasserstoff sind,

herbizid wirksam und gleichzeitig für eine Reihe von Kulturpflanzen verträglich und selektiv sind.

Kg/GS

$R^1$ in Formel I bedeutet Wasserstoff oder $C_1$-$C_4$-Alkyl, wie Methyl, Ethyl, n-Propyl, i-Butyl, vorzugsweise Methyl oder Ethyl. $R^2$ in Formel I bedeutet $C_1$-$C_4$-Alkyl, $C_1$-$C_4$-Alkoxy, $C_2$-$C_4$-Alkenyl, $C_2$-$C_4$-Alkinyl oder $C_3$-$C_7$-Cycloalkyl, beispielsweise Methyl, Ethyl, n-Propyl, Isopropyl, t-Butyl, Methoxy, Ethoxy, Vinyl, Allyl, Ethinyl, Prop-1-inyl, Propargyl, But-2-inyl, But-1-in-3-yl, But-1-in-4-yl, 3-Methyl-but-1-in-3-yl, Cyclopropyl, Cyclobutyl, Cyclopentyl, Cyclohexyl. Bevorzugt sind Methyl und Methoxy.

X in Formel I bedeutet Wasserstoff, Halogen, $C_1$-$C_6$-Alkoxy, vorzugsweise $C_1$-$C_4$-Alkoxy, $C_1$-$C_6$-Halogenalkoxy, vorzugsweise $C_1$-$C_4$-Halogenalkoxy, wobei als Halogensubstituenten insbesondere Fluor und/oder Chlor in Betracht kommen, $C_1$-$C_6$-Alkyl, vorzugsweise $C_1$-$C_4$-Alkyl, $C_1$-$C_6$-Halogenalkyl, vorzugsweise $C_1$-$C_4$-Halogenalkyl, wobei als Halogensubstituenten insbesondere Fluor und/oder Chlor in Betracht kommen, $C_3$-$C_6$-Cycloalkyl, $C_1$-$C_6$-Alkylthio, vorzugsweise $C_1$-$C_4$-Alkylthio, Nitro, Cyano, Benzyloxy, gegebenenfalls durch Halogen, wie Chlor, substituiertes Phenyl oder gegebenenfalls durch Halogen, wie Chlor und/oder Halogenmethyl, wie Trifluormethyl, substituiertes Phenoxy, beispielsweise Fluor, Chlor, Brom oder Iod, vorzugsweise Fluor, Chlor oder Brom, Methoxy, Ethoxy, Difluormethoxy, 3,3,3,2,1,1-Hexafluor-n-propoxy, 2,1,1-Trifluor-2-chlor-ethoxy, Methyl, Ethyl, t-Butyl, Trifluormethyl, Cyclohexyl, Methylthio, Ethylthio, Nitro, Cyano, Benzyloxy, Phenyl, 4-Chlorphenyl, 4-Trifluormethylphenyl, 2-Chlor-4-trifluormethylphenoxy, Phenoxy, 4-Chlorphenoxy.

Bevorzugte Thiazolylharnstoffe der Formel I sind solche, bei denen $R^1$ $C_1$-$C_4$-Alkyl, vorzugsweise Methyl, und $R^2$ $C_1$-$C_4$-Alkyl, vorzugsweise Methyl, oder $C_1$-$C_4$-Alkoxy, vorzugsweise Methoxy, bedeuten. Weiterhin bevorzugte Thiazolylharnstoffe der Formel I sind solche, bei denen X $C_1$-$C_6$-Halogenalkoxy, insbesondere durch Fluor und/oder Chlor substituiertes Alkoxy oder Halogen, insbesondere Fluor, Chlor oder Brom, bedeuten.

Man erhält die Thiazolylharnstoffe der Formel I durch Umsetzung von substituierten Aminen der Formel II

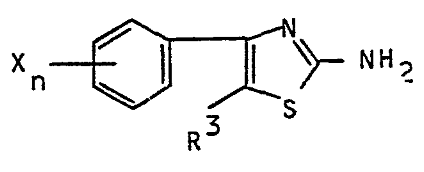 (II),

in der $R^3$, X und n die obengenannten Bedeutungen haben, mit

a) Phosgen, Oxazolylchlorid oder Diphosgen und anschließend mit einem Amin der Formel III

$$HNR^1R^2 \qquad (III),$$

in der $R^1$ und $R^2$ die obengenannten Bedeutungen haben oder

b) mit einem Carbamidsäurehalogenid der Formel IV

$$Hal\text{-}CO\text{-}NR^1R^2 \qquad (IV),$$

in der $R^1$ und $R^2$ die obengenannten Bedeutungen haben und Hal für Halogen steht.

Thiazolylharnstoffe der Formel I, bei denen $R^2$ $C_1$-$C_4$-Alkyl bedeutet und $R^3$, X und n die obengenannten Bedeutungen haben, können auch durch Umsetzung eines substituierten Amins der Formel II mit einem Isocyanat der Formel V

$$R^2NCO \qquad (V),$$

in der $R^2$ $C_1$-$C_4$-Alkyl bedeutet, erhalten werden.

Die oben beschriebenen Umsetzungen werden vorzugsweise in Gegenwart geeigneter Lösungs- oder Verdünnungsmittel durchgeführt. Als solche kommen praktisch alle inerten organischen Solventien in Betracht.

Hierzu gehören insbesondere aromatische und aliphatische Kohlenwasserstoffe, wie Ligroin, Benzin, Toluol, Pentan, Hexan, Cyclohexan, Petrolether, aromatische und aliphatische Halogenkohlenwasserstoffe, wie Methylenchlorid, Chloroform, Tetrachlorkohlenstoff, 1,1- und 1,2-Dichlorethan, 1,1,1- und 1,1,2-Trichlorethan, Chlorbenzol, o-, m-, p-Dichlorbenzol, o-, m-, p-Chlortoluol, aromatische und aliphatische Nitrokohlenwasserstoffe, wie Nitrobenzol, Nitroethan, o-, m-, p-Nitrotoluol, Nitrile, wie Acetonitril, Butyronitril, Isobutyronitril, Ether, wie Diethylether, Di-n--propylether, Tetrahydrofuran, Dioxan, Ester, wie Acetessigester, Ethylacetat, Isobutylacetat, ferner Ketone, wie Aceton, Methylethyl-, Methylisopropyl- und Methylisobutylketon, außerdem Amide, wie Methylformamid und Dimethylformamid. Auch Gemische dieser Lösungsmittel können verwendet werden.

Die Umsetzung der Amine der Formel II mit den Aminen der Formel III läßt sich sehr gut durchführen, indem man das substituierte Amin der Formel II

zunächst mit einem Überschuß Phosgen , Diphosgen oder Oxalylchlorid in das entsprechende Isocyanat überführt (Houben-Weyl, Methoden der organ. Chemie, Bd. 8, S. 122 und 123, 1952; Methodicum Chimicum, Bd. 6, S. 780, 1974) und anschließend mit mindestens der äquivalenten Menge Amin der Formel III gegebenenfalls in Gegenwart eines Lösungsmittels bei einer Temperatur von -10°C bis +150°C, vorzugsweise von +20°C bis +120°C, kontinuierlich oder diskontinuierlich umsetzt.

Zur Durchführung der Reaktion des substituierten Amins der Formel II mit dem Carbamidsäurehalogenid der Formel IV wird das Carbamidsäurehalogenid in einem Überschuß von bis zu 50 % (Mol% oder Gew.%), gegebenenfalls in Gegenwart eines Lösungsmittels und gegebenenfalls in Gegenwart eines Säurebinders, bei einer Temperatur von -10 bis +150°C, vorzugsweise von 20 bis 120°C, kontinuierlich oder diskontinuierlich umgesetzt (Houben-Weyl, Methoden der organischen Chemie, Bd. 8, S. 160 und 161, 1951).

Als Säurebinder können alle üblichen Säurebindemittel verwendet werden. Hierzu gehören vorzugsweise Alkalihydroxide, Alkalicarbonate, Alkalialkoholate und tertiäre organische Basen. Als besonders geeignet seien im einzelnen genannt: Natriumhydroxid, Natriumhydrogencarbonat, Natriummethylat, Triethylamin, Pyridin, Trimethylamin, alpha-, beta-Picolin, Lutidin, N,N-Dimethylanilin, N,N-Dimethylcyclohexylamin, Chinolin, Tri-n-propylamin, Tri-n-butylamin und Acridin, N,N-Dimethyl- und N,N-Diethylpiperazin, N-Methyl- und N-Ethylpiperidin sowie N-Methyl- und N-Ethylpyrrolidin.

Bei der Verfahrensvariante, die auf der Umsetzung von Amin mit Isocyanat beruht, wird das substituierte Amin der Formel II mit einem Überschuß von bis zu 50 % (Mol% oder Gew.%) Isocyanat der Formel V, gegebenenfalls in Gegenwart eines Lösungsmittels bei einer Temperatur von -10 bis +150°C, vorzugsweise 20 bis 120°C, kontinuierlich oder diskontinuierlich umgesetzt (Houben-Weyl, Methoden der organischen Chemie, Bd. 8, S. 157 bis 159, 1952).

Die Isolierung der Endprodukte erfolgt bei allen Verfahrensvarianten entweder bei aus dem Reaktionsgemisch ausgefallenen Stoffen durch Absaugen und anschließende Reinigung durch Umkristallisieren bzw. Chromatographieren oder durch Einengen des Reaktionsgemisches unter vermindertem Druck zur Trockne und Reinigung des Rückstandes durch Umkristallisieren bzw. Chromatographieren.

Die als Ausgangsstoffe verwendbaren substituierten Amine der Formel II sind zum Teil neu. Sie können aber durch an sich bekannte Verfahren hergestellt werden (J. Am Chem. Soc. 67, 2242 (1945)).

Die folgenden Beispiele erläutern die Herstellung der Thiazolylharnstoffe der Formel I.

Beispiel 1

30 g 4-Methoxyacetophenon wurden mit 33,5 g Thioharnstoff versetzt. Man gab 56 g Jod zu und hielt 5 Stunden bei 100°C. Das Reaktionsgemisch wurde auf Wasser ausgetragen, gepulvert und mehrfach mit Ether, dann mit Ammoniaklösung und Wasser gewaschen und getrocknet. Man erhielt 37,8 g 4-(4-Methoxyphenyl)-2-amino-thiazol vom Schmelzpunkt (Fp) 194-205°C.

Dann wurden 6,2 g 4-(4-Methoxyphenyl)-2-amino-thiazol, 80 ml Toluol und 2 Tropfen Dibutylzinndiacetat vorgelegt. Bei 50°C tropfte man dann langsam 2,9 g Methylisocyanat zu und hielt insgesamt 12 Stunden bei 50°C. Der Festkörper wurde abgesaugt, mit Toluol gewaschen und getrocknet. Man erhielt 5,5 g N-Methyl-N'-[4-(4-methoxyphenyl)-thiazol-2-yl]-harnstoff vom Schmelzpunkt 200-208°C. Aus der Mutterlauge ließen sich weitere 2,5 g gewinnen (Verbindung Nr. 73).

Beispiel 2

Es wurden 10 g 4-(4-Methoxyphenyl)-2-amino-thiazol 100 ml Aceton und 7 g N-Methylpiperidin vorgelegt. Man tropfte 6,9 g Methoxymethylcarbamoylchlorid bei 30°C über 3 Stunden zu und hielt 4 Stunden bei 30°C. Der Festkörper wurde abgesaugt. Die Aceton-Lösung wurde in Wasser eingerührt, abgesaugt und getrocknet. Man erhielt 12,9 g N-Methyl-N-methoxy-N'-[4-(4-methoxyphenyl)-thiazol-2-yl]- harnstoff vom Schmelzpunkt 125-128°C, der sich nach Waschen mit verd. Aceton auf 133-134°C erhöhte (Verbindung Nr. 10).

Beispiel 3

26,8 g 4-Methylacetophenon und 33,5 g Thioharnstoff wurden auf einmal mit 56 g Jod versetzt. Man hielt 3 Stunden bei 100°C und trug heiß auf 500 ml Eiswasser aus. Der Festkörper wurde abgesaugt, gut mit Ether, verdünnter Ammoniaklösung und Wasser gewaschen und dann getrocknet. Man erhielt 32,2 g 4-(4-Methylphenyl)-2-amino-thiazol vom Schmelzpunkt 131-135°C.

11,6 g 4-(4-Methylphenyl)-2-amino-thiazol wurden in 100 ml Chlorbenzol mit 23 g Oxalylchlorid versetzt, langsam zum Sieden unter Rückfluß gebracht und bis zum Ende der Gasentwicklung unter Rückfluß gehalten. Die Lösung wurde kalt in 60 ml Dimethylaminlösung getropft und 2 Stunden nachgerührt, der Niederschlag wurde abgesaugt, mit Wasser und Petrolether ge-

waschen und getrocknet. Man erhielt 7,2 g N,N-Dimethyl-N'-[4-(4-methylphenyl)-thiazol-2-yl]-harnstoff vom Schmelzpunkt 145-151°C (Verbindung Nr. 51).

Beispiel 4

Es wurden 5,7 g 4-(4-Methylphenyl)-2-amino-thiazol, 50 ml Toluol und 7 ml Pyridin vorgelegt. Man tropfte 4 g N-Methyl-N-methoxycarbamoylchlorid zu und hielt 8 Stunden bei 50°C. Der Niederschlag wurde abgesaugt und verworfen, die Mutterlauge mit Wasser, verd. Salzsäure und Wasser gewaschen, getrocknet und eingeengt. Man erhielt 4,3 g N-Methyl-N-methoxy-N'-[4-(4-methylphenyl)-thiazol-2-yl]- harnstoff vom Brechungsindex $n_D^{25}$ = 1,6072 (Verbindung Nr. 12).

Analog wurden die folgenden Thiazolylharnstoffe der Formel I hergestellt:

| Verbindung Nr. | X | $R^3$ | $R^2$ | $R^1$ | Fp [°C]/$n_D$/ $\nu_{CO}$ |
|---|---|---|---|---|---|
| 1 | 4-$CF_3$ | H | $CH_3$ | H | 254-258 |
| 2 | 4-(4-Trifluor-methyl-2-chlor--phenoxy) | H | $CH_3$ | H | 200-206 |
| 3 | H | $CH_3$ | $CH_3$ | H | 256-260 |
| 4 | 4-$OCH_3$ | H | $CH_3$ | $CH_3$ | 95-104 |
| 5 | 4-(4-Trifluor-methyl-2-chlor--phenoxy) | H | $CH_3O$ | $CH_3$ | zähe Masse* |
| 6 | 4-$NO_2$ | H | $CH_3O$ | $CH_3$ | 190-195 |
| 7 | 4-$CF_3$ | H | $CH_3O$ | $CH_3$ | 145-148 |
| 8 | 4-$CH_3$ | H | $CH_3$ | H | 175-180 |
| 9 | 4-$OCH_3$ | H | $CH(CH_3)C{\equiv}CH$ | $CH_3$ | |
| 10 | 4-$OCH_3$ | H | $CH_3O$ | $CH_3$ | 128-132 |
| 11 | H | $CH_3$ | $CH_3O$ | $CH_3$ | $n_D^{25}$: 1,6094 |
| 12 | 4-$CH_3$ | H | $CH_3O$ | $CH_3$ | $n_D^{25}$: 1,6072 |
| 13 | 4-Cl | H | $CH_3O$ | $CH_3$ | 119-121 |
| 14 | 4-Cl | H | $CH_3$ | $CH_3$ | 139-142 |
| 15 | 3-Cl | H | $CH_3$ | H | |
| 16 | 3,4-$Cl_2$ | H | $CH_3$ | $CH_3$ | |
| 17 | 3,4-$Cl_2$ | H | $OCH_3$ | $CH_3$ | 105-108 |
| 18 | 4-F | H | $CH_3$ | H | |
| 19 | 4-F | H | $CH_3$ | $CH_3$ | |
| 20 | 4-F | H | $CH_3O$ | $CH_3$ | 123-125 |

*$^1$H-NMR: 3,2 ppm (3 Prot.) 3,75 ppm (3 Prot.) 7 ppm (1 Prot., Dubl.)
7,1 ppm (2 Prot, Dubl.) 7,5 ppm (1 Prot., Dubl.)
7,75 ppm (1 Prot. Sing.)
7,85 ppm (2 Prot. Dubl.) 9,25 ppm (1 Prot., breit)

| Verbindung Nr. | X | $R^3$ | $R^2$ | $R^1$ | Fp [°C]/$n_D$/ $\nu_{CO}$ |
|---|---|---|---|---|---|
| 21 | 3-Br | H | $CH_3$ | H | 258-261 |
| 22 | 3-Br | H | $CH_3$ | $CH_3$ | 137-141 |
| 23 | 3-Br | H | $CH_3O$ | $CH_3$ | $\nu_{CO}$ = 1685 $cm^{-1}$ |
| 24 | H | $CH_3$ | $CH_3$ | H | |
| 25 | H | H | $CH_3$ | $CH_3$ | 50- 74 |
| 26 | H | H | $CH_3O$ | $CH_3$ | $\nu_{CO}$ = 1685 $cm^{-1}$ |
| 27 | 3-$OCH_3$ | H | $CH_3$ | H | |
| 28 | 3-$OCH_3$ | H | $CH_3$ | $CH_3$ | |
| 29 | 3-$OCH_3$ | H | $CH_3O$ | $CH_3$ | $\nu_{CO}$ = 1691 $cm^{-1}$ |
| 30 | 4-$OC_2H_5$ | H | $CH_3$ | H | |
| 31 | 4-$OC_2H_5$ | H | $CH_3$ | $CH_3$ | |
| 32 | 4-$OC_2H_5$ | H | $CH_3O$ | $CH_3$ | 153-156 |
| 33 | 4-$OCH_3$ | $CH_3$ | $CH_3$ | H | |
| 34 | 4-$OCH_3$ | $CH_3$ | $CH_3$ | $CH_3$ | |
| 35 | 4-$OCH_3$ | $CH_3$ | $CH_3O$ | $CH_3$ | $n_D^{25}$: 1,6008 |
| 36 | 4-$OC_6H_5$ | H | $CH_3$ | H | |
| 37 | 4-$OC_6H_5$ | H | $CH_3$ | $CH_3$ | |
| 38 | 4-$OC_6H_5$ | H | $CH_3O$ | $CH_3$ | $n_D^{25}$: 1,4934 |
| 39. | 4-$OCH_2C_6H_5$ | H | $CH_3$ | H | 145-151 |
| 40 | 4-$OCH_2C_6H_5$ | H | $CH_3$ | $CH_3$ | |
| 41 | 4-$OCH_2C_6H_5$ | H | $CH_3O$ | $CH_3$ | 165-170 |
| 42 | 2-Cl | H | $CH_3$ | H | |
| 43 | 2-Cl | H | $CH_3$ | $CH_3$ | |
| 44 | 2-Cl | H | $CH_3O$ | $CH_3$ | $n_D^{25}$: 1,5988 |
| 45 | 4-$OCHF_2$ | H | $CH_3$ | H | |
| 46 | 4-$OCHF_2$ | H | $CH_3$ | $CH_3$ | |
| 47 | 4-$OCHF_2$ | H | $CH_3O$ | $CH_3$ | 84- 88 |
| 48 | 4-(4-Chlorphenoxy) | H | $CH_3$ | H | |
| 49 | 4-(4-Chlorphenoxy) | H | $CH_3$ | $CH_3$ | |
| 50 | 4-(4-Chlorphenoxy) | H | $CH_3O$ | $CH_3$ | |
| 51 | 4-$CH_3$ | H | $CH_3$ | $CH_3$ | |
| 52 | 4-$SCH_3$ | H | $CH_3$ | H | |
| 53 | 4-$SCH_3$ | H | $CH_3$ | $CH_3$ | |
| 54 | 4-$SCH_3$ | H | $CH_3$ | $CH_3$ | 159-162 |
| 55 | 3-Cl, 4-$OCH_3$ | H | $CH_3$ | H | |
| 56 | 3-Cl, 4-$OCH_3$ | H | $CH_3$ | $CH_3$ | |
| 57 | 3-Cl, 4-$OCH_3$ | H | $CH_3O$ | $CH_3$ | 119-127 |
| 58 | 4-cyclo-$C_6H_{11}$ | H | $CH_3$ | H | |
| 59 | 4-cyclo-$C_6H_{11}$ | H | $CH_3$ | $CH_3$ | |
| 60 | 4-cyclo-$C_6H_{11}$ | H | $CH_3O$ | $CH_3$ | 119-127 |
| 61 | 4-O-$CF_2CHF$-$CF_3$ | H | $CH_3$ | H | 106-110 |

| Verbindung Nr. | X | $R^3$ | $R^2$ | $R^1$ | Fp [°C]/$n_D$/ $\nu_{CO}$ |
|---|---|---|---|---|---|
| 62 | 4-O-$CF_2$CHF-$CF_3$ | H | $CH_3$ | $CH_3$ | |
| 63 | 4-O-$CF_2$CHF-$CF_3$ | H | $CH_3$ | $OCH_3$ | 88-93 |
| 64 | 4-O-$CF_2$CHClF | H | $CH_3$ | H | |
| 65 | 4-O-$CF_2$CHClF | H | $CH_3$ | $CH_3$ | |
| 66 | 4-O-$CF_2$CHClF | H | $CH_3$ | $OCH_3$ | |
| 67 | 4-$OCH_3$, 2-Cl | H | $CH_3$ | H | |
| 68 | 4-$OCH_3$, 2-Cl | H | $CH_3$ | $CH_3$ | |
| 69 | 4-$OCH_3$, 2-Cl | H | $CH_3$ | $OCH_3$ | 115-118 |
| 70 | 2-$OCH_3$, 4-Cl | H | $CH_3$ | H | |
| 71 | 2-$OCH_3$, 4-Cl | H | $CH_3$ | $CH_3$ | |
| 72 | 2-$OCH_3$, 4-Cl | H | $CH_3$ | $OCH_3$ | $\nu_{CO}$ = 1684 $cm^{-1}$ |
| 73 | 4-$OCH_3$, | H | H | $CH_3$ | 200-208 |
| 74 | 4-$CF_3$ | H | $CH_3$ | $CH_3$ | 202-210 |
| 75 | 2,4-$Cl_2$ | H | $CH_3$ | $OCH_3$ | 91-93 |
| 76 | 2,5-$Cl_2$ | H | $CH_3$ | $OCH_3$ | |
| 77 | 2,3,4-$Cl_3$ | H | $CH_3$ | $OCH_3$ | |
| 78 | 2-F | H | $CH_3$ | $OCH_3$ | |
| 79 | 4-Br | H | $CH_3$ | $OCH_3$ | |
| 80 | 2,4-($F_2$HC-O-$)_2$ | H | $CH_3$ | $OCH_3$ | |
| 81 | 2-$CF_3$ | H | $CH_3$ | $OCH_3$ | |
| 82 | 3-$CF_3$ | H | $CH_3$ | $OCH_3$ | |
| 83 | 4-tert-$C_4H_9$ | H | $CH_3$ | $OCH_3$ | |
| 84 | 2,4,6-$(CH_3)_3$ | H | $CH_3$ | $OCH_3$ | |
| 85 | 4-$OCF_3$ | H | $CH_3$ | $OCH_3$ | |
| 86 | 3-Cl, 4-(4-Cl-$C_6H_4$-O) | H | $CH_3$ | $OCH_3$ | 120-125 |
| 87 | 2,4-$F_2$ | H | $CH_3$ | $OCH_3$ | |
| 88 | 2,3,5-$Cl_3$ | H | $CH_3$ | $OCH_3$ | |
| 89 | 2-$OCH_3$, 3,5-$Cl_2$ | H | $CH_3$ | $OCH_3$ | |
| 90 | 2-$OCH_3$, 4-$CH_3$ | H | $CH_3$ | $OCH_3$ | |
| 91 | 4-$OCH_3$, 2-$CH_3$ | H | $CH_3$ | $OCH_3$ | |
| 92 | 4-$OCHF_2$, 2-$CH_3$ | H | $CH_3$ | $OCH_3$ | |
| 93 | 2-$OCHF_2$, 4-$CH_3$ | H | $CH_3$ | $OCH_3$ | |
| 94 | 4-$OCHF_2$, 2-Cl | H | $CH_3$ | $OCH_3$ | |
| 95 | 4-Cl, 2-$OCHF_2$ | H | $CH_3$ | $OCH_3$ | |
| 96 | 3,5-$Cl_2$ | H | $CH_3$ | $OCH_3$ | |
| 97 | 2,6-$Cl_2$ | H | $CH_3$ | $OCH_3$ | |

Die Thiazolylharnstoffe der Formel I können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittleren Naphthalinen oder Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenon, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attapulgus Clay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten zwischen 0,1 und 95 Gewichtsprozent, vorzugsweise zwischen 0,5 und 90 Gewichtsprozent, Wirkstoff.

Beispiele für Formulierungen sind:

I. Man vermischt 90 Gewichtsteile der Verbindung Nr. 2 mit 10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 8 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gewichtsprozent des Wirkstoffs enthält.

III. 20 Gewichtsteile der Verbindung Nr. 1 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteile des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

IV. 20 Gewichtsteile des Wirkstoffs Nr. 1 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

V. 20 Gewichtsteile des Wirkstoffs Nr. 2 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

VI. 3 Gewichtsteile des Wirkstoffs Nr. 9 werden mit 97 Gewichtsteilen feinteiligem Kaolin vermischt. Man erhält auf diese Weise ein Stäubemittel, das 3 Gewichtsprozent des Wirkstoffs enthält.

VII. 30 Gewichtsteile des Wirkstoffs Nr. 9 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Teile des Wirkstoffs Nr. 6 werden mit 2 Teilen Calciumsalz der Dodecylbenzolsulfonsäure, 8 Teilen Fettalkohol-polyglykolether, 2 Teilen Natriumsalz eines Phenol-Harnstoff-Formaldehyd-Kondensates und 68 Teilen eines paraffinischen Mineralöls innig vermischt. Man erhält eine stabile ölige Dispersion.

Die Applikation der Wirkstoffe bzw. der Mittel kann im Vorauflaufverfahren oder im Nachauflaufverfahren erfolgen. Sind die Wirkstoffe bei Nachauflaufanwendung für gewisse Kulturpflanzen weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit, Zielpflanzen und Wachstumsstadium 0,1 bis 5 kg/ha, vorzugsweise 0,125 bis 1,5 kg/ha.

Die Wirkung der Thiazolylharnstoffe der Formel I auf das Pflanzenwachstum läßt sich durch Gewächshausversuche zeigen:

Als Kulturgefäße dienen Plastikblumentöpfe mit 300 $cm^3$ Inhalt und lehmigem Sand mit etwa 1,5 % Humus als Substrat. Die Samen der Testpflanzen werden nach Arten getrennt flach eingesät. Dann werden die Testpflanzen je nach Wuchsform bis zu einer Wuchshöhe von 3 bis 15 cm angezogen und mit Wirkstoff bzw. herbizidem Mittel behandelt. Die Soja- und Reispflanzen werden in einem mit Torfmull (peat) angereicherten Substrat angezogen. Es werden entweder direkt gesäte und in den gleichen Gefäßen aufgewachsene Pflanzen ausgewählt, oder sie werden erst als Keimpflanzen getrennt angezogen und einige Tage vor der Behandlung in die Versuchsgefäße verpflanzt. Die Aufwandmengen für die Nachauflaufbehandlung variieren zwischen 0,25 und 3,0 kg Wirkstoff/ha.

Die Versuchsgefäße werden im Gewächshaus aufgestellt, wobei für wärmeliebende Arten wärmere Bereiche (20 bis 35°C) und für solche gemäßigter Klimate 10 bis 25°C bevorzugt werden. Die Versuchsperiode erstreckt sich über 2 bis 4 Wochen. Während dieser Zeit werden die Pflanzen gepflegt und ihre Reaktion auf die einzelnen Behandlungen wird ausgewertet. Bewertet wird nach einer Skala von 0 bis 100. Dabei bedeutet 100 kein Aufgang der Pflanzen bzw. völlige Zerstörung zumindest der oberirdischen Teile.

Die in den Gewächshausversuchen verwendeten Pflanzen setzen sich aus folgenden Arten zusammen:

Abutilon theophrasti (Chinesischer Hanf), Amaranthus retroflexus (Zurückgekrümmter Fuchsschwanz), Cassia tora, Chenopodium album (Weißer Gänsefuß), Echinochloa crus-galli (Hühnerhirse), Euphorbia geniculata (Südamerik. Wolfsmilchart), Glycine max (Sojabohnen), Gossypium hirsutum (Baumwolle), Ipomoea spp. (Prunkwindearten), Lolium multiflorum (Ital. Raygras), Mercurialis annua (Einjähriges Bingelkraut), Oryza sativa (Reis), Sesbania exaltata (Turibaum), Sida spinosa, Sorghum bicolor (Mohrenhirse, Kulturhirse), Triticum aestivum (Weizen).

Vergleichsmittel sind Herbizide, die den aus der GB-A-1 131 207 bekannten Wirkstoff N-[4-(4-Chlorphenyl)-thiazol-2-yl]-N'-methyl-harnstoff (im folgenden als "A" bezeichnet) enthalten.

Bei Nachauflaufanwendung von 3,0 kg Wirkstoff/ha zeigen beispielsweise die Verbindungen Nr. 5, 7, 13 und 14 eine beachtliche herbizide Aktivität gegen breitblättrige und grasartige Pflanzen.

Die Verbindungen Nr. 7 und 13 bekämpfen mit 0,25 kg Wirkstoff/ha unerwünschte Pflanzen wesentlich besser als das Vergleichsmittel A. Dasselbe gilt für Verbindung Nr. 47 bei einer Aufwandmenge von 1,0 kg Wirkstoff/ha. Die Verbindungen Nr. 12 und 14 bekämpfen mit 0,5 kg Wirkstoff/ha spezielle breitblättrige Unkräuter, wie Sesbania exaltata.

Wie das Vergleichsmittel A ist auch Verbindung Nr. 20 für Baumwolle gut verträglich. Die herbizide Aktivität der neuen Verbindung gegen breitblättrige Unkräuter in dieser Kultur ist dem Mittel A jedoch deutlich überlegen.

Mit einer Aufwandmenge von 0,25 kg Wirkstoff/ha lassen sich wichtige Unkräuter in Sojakulturen mit der Verbindung Nr. 75 erfolgreich bekämpfen, ohne die Kulturpflanze zu schädigen. Mit Vergleichsmittel A ist kein ausreichender Bekämpfungserfolg zu erzielen.

In Anbetracht der Verträglichkeit und der Vielzahl der Applikationsmethoden können die Thiazolylharnstoffe der Formel I noch in einer weiteren Zahl von Kulturpflanzen zur Beseitigung unerwünschter Wildgräser oder grasartiger Kulturpflanzen, sofern sie an gewissen Standorten unerwünscht sind, eingesetzt werden.

In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var, silvestris | Rübsen |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum Gossypium herbaceum Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactua sativa | Kopfsalat |

| Botanischer Name | Deutscher Name |
|---|---|
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Vaccinium corymbosum | Kulturheidelbeere |
| Vaccinium vitis-idaea | Preißelbeere |

| Botanischer Name | Deutscher Name |
| --- | --- |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die Thiazolylharnstoffe der Formel I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone,

2,6-Dinitroaniline, N-Phenylcarbamate, Thiolcarbamate, Halogencarbonsäuren, Triazine, Amide, Harnstoffe anderer Struktur, Diphenylether, Triazinone, Uracile, Benzofuranderivate, Cyclohexan-1,3-dionderivate und andere herbizide Wirkstoffe in Betracht.

Außerdem kann es von Nutzen sein, die Thiazolylharnstoffe der Formel I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Bekämpfung von Ernährungs- und Spurenelementmängeln eingesetzt werden.

Patentansprüche

1. Thiazolylharnstoffe der Formel I

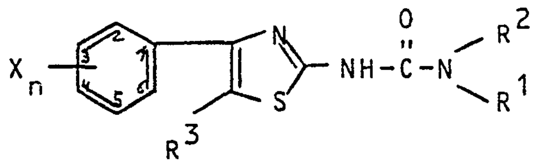
(I),

in der

$R^1$ Wasserstoff oder $C_1$-$C_4$-Alkyl,

$R^2$ $C_1$-$C_4$-Alkyl, $C_1$-$C_4$-Alkoxy, $C_2$-$C_4$-Alkenyl, $C_2$-$C_4$-Alkinyl oder $C_3$-$C_7$-Cycloalkyl,

$R^3$ Wasserstoff oder $C_1$-$C_4$-Alkyl,

X Wasserstoff, Halogen, $C_1$-$C_6$-Alkoxy, $C_1$-$C_6$-Halogenalkoxy, $C_1$-$C_6$-Alkyl, $C_1$-$C_6$-Halogenalkyl, $C_3$-$C_6$-Cycloalkyl, $C_1$-$C_6$-Alkylthio, Nitro, Cyano, Benzyloxy, gegebenenfalls durch Halogen substituiertes Phenyl oder gegebenenfalls durch Halogen und/oder Halogenmethyl substituiertes Phenoxy und

n 1, 2 oder 3 bedeuten,

mit der Maßgabe, daß X nicht Wasserstoff, Chlor oder Methoxy in 4-Position oder Chlor in 3- und 4-Position bedeutet, wenn $R^1$ Methyl, $R^2$ und $R^3$ Wasserstoff sind.

2. Thiazolylharnstoffe der Formel I gemäß Anspruch 1, dadurch gekennzeichnet, daß $R^1$ Methyl und $R^2$ Methyl oder Methoxy bedeuten.

3. Thiazolylharnstoffe der Formel I gemäß Anspruch 1, dadurch gekennzeichnet, daß X $C_1$-$C_6$-Halogenalkoxy bedeutet.

4. Thiazolylharnstoffe der Formel I gemäß Anspruch 1, dadurch gekennzeichnet, daß X Halogen bedeutet.

5. Verfahren zur Herstellung von Thiazolylharnstoffen der Formel I gemäß Anspruch 1, dadurch gekennzeichnet, daß man ein substituiertes Amin der Formel II

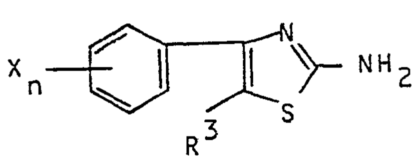
(II),

in der $R^3$, X und n die im Anspruch 1 genannten Bedeutungen haben,

a) mit Phosgen, Oxalylchlorid oder Diphosgen und anschließend mit einem Amin der Formel III

$$HNR^1R^2 \quad (III),$$

in der $R^2$ und $R^1$ die im Anspruch 1 genannten Bedeutungen haben, oder

b) mit einem Carbamidsäurehalogenid der Formel IV

$$Hal\text{-}CO\text{-}NR^1R^2 \quad (IV),$$

in der $R^1$ und $R^2$ die im Anspruch 1 genannten Bedeutungen haben und Hal für Halogen steht, umsetzt.

6. Verfahren zur Herstellung von Thiazolylharnstoffen der Formel I gemäß Anspruch 1, wobei $R^2$ $C_1$-$C_4$-Alkyl bedeutet und $R^3$ und $X_n$ die im Anspruch 1 genannten Bedeutungen haben, dadurch gekennzeichnet, daß man ein substituiertes Amin der Formel II gemäß Anspruch 5 mit einem Isocyanat der Formel V

$$R^2NCO \quad (V),$$

in der $R^2$ $C_1$-$C_4$-Alkyl bedeutet, in an sich üblicher Weise umsetzt.

7. Herbizid, enthaltend einen Thiazolylharnstoff der Formel I gemäß Anspruch 1.

8. Herbizid, enthaltend inerte Zusatzstoffe und einen Thiazolylharnstoff der Formel I gemäß Anspruch 1.

9. Herbizid gemäß Anspruch 8, dadurch gekennzeichnet, daß es einen Thiazolylharnstoff der Formel I enthält, wobei $R^1$ Methyl und $R^2$ Methyl oder Methoxy bedeuten.

10. Herbizid gemäß Anspruch 8, dadurch gekennzeichnet, daß es einen Thiazolylharnstoff der Formel I enthält, wobei X $C_1$-$C_6$-Halogenalkoxy bedeutet.

11. Herbizid gemäß Anspruch 8, dadurch gekennzeichnet, daß es einen Thiazolylharnstoff der Formel I enthält, wobei X Halogen bedeutet.

12. Verfahren zur Herstellung eines herbiziden Mittels gemäß Anspruch 7, dadurch gekennzeichnet, daß man inerte Zusatzstoffe mit 0,1 bis 95 Gew.% eines Thiazolylharnstoffs der Formel I gemäß Anspruch 1 vermischt.

13. Verfahren zur Bekämpfung von unerwünschtem Pflanzenwuchs, dadurch gekennzeichnet, daß man die unerwünschten Pflanzen oder von unerwünschtem Pflanzenwuchs freizuhaltende Flächen mit einer herbizid wirksamen Menge eines Thiazolylharnstoffs der Formel I gemäß Anspruch 1 behandelt.